# EUROPEAN PATENT SPECIFICATION

(11) **EP 3 505 213 B1**
(45) Date of publication and mention of the grant of the patent: **30.11.2022**
(21) Application number: 16856453.2
(22) Date of filing: 23.08.2016
(51) Int. Cl.: A61M 25/00, A61M 39/10, B29C 65/58, F16L 13/10

(54) **JOINT STRUCTURE AND CATHETER HAVING SAID JOINT STRUCTURE**
VERBINDUNGSSTRUKTUR UND KATHETER MIT BESAGTER VERBINDUNGSSTRUKTUR
STRUCTURE D'ARTICULATION ET CATHÉTER PRÉSENTANT LADITE STRUCTURE D'ARTICULATION

(43) Date of publication of application: 03.07.2019
(73) Proprietor: ASAHI INTECC CO., LTD., Seto-shi, Aichi 489-0071 (JP)
(72) Inventor: SHIMIZU, Yusuke, Seto-shi, Aichi 489-0071 (JP)
(74) Representative: Winter, Brandl - Partnerschaft mbB
(86) International application number: PCT/JP2016/074490
(87) International publication number: WO 2018/037475

(56) References cited:
- EP-A1- 2 135 634
- WO-A1-97/14466
- WO-A1-97/14466
- WO-A1-98/56447
- WO-A1-2009/125575
- WO-A1-2014/099899
- JP-A- H0 884 776
- JP-A- H09 512 445
- JP-A- H11 239 617
- JP-A- 2013 533 767
- JP-A- 2016 507 269
- US-A- 4 755 408
- US-A- 5 160 559
- US-A- 5 584 821
- US-A- 5 584 821
- US-A- 6 103 037
- US-A- 6 103 037
- US-A1- 2015 374 252

## Description

### TECHNICAL FILED

The present invention relates to a junction structure for joining corresponding end portions of two tubular members, and a catheter having the junction structure.

### BACKGROUND ART

A catheter to be inserted into a lumen in the body such as blood vessel, ureter, and the like of a patient at the time of procedure generally includes a tube member, a front end tip joined to the front end of the tube member, and a connector joined to the base end of the tube member.

Further, in such a catheter, multiple tubular members having different hardnesses are provided such that a tubular member having a lower hardness is sequentially joined in series toward the front end in order to gradually increase flexibility toward the front end.

For a tube member of a catheter manufactured by sequentially joining multiple tubular members as described above, the joined tubular members need to have a joining strength sufficient for preventing detachment of the joined tubular members even when the catheter inserted into a body lumen of a patient is bent along the curvature of the body lumen.

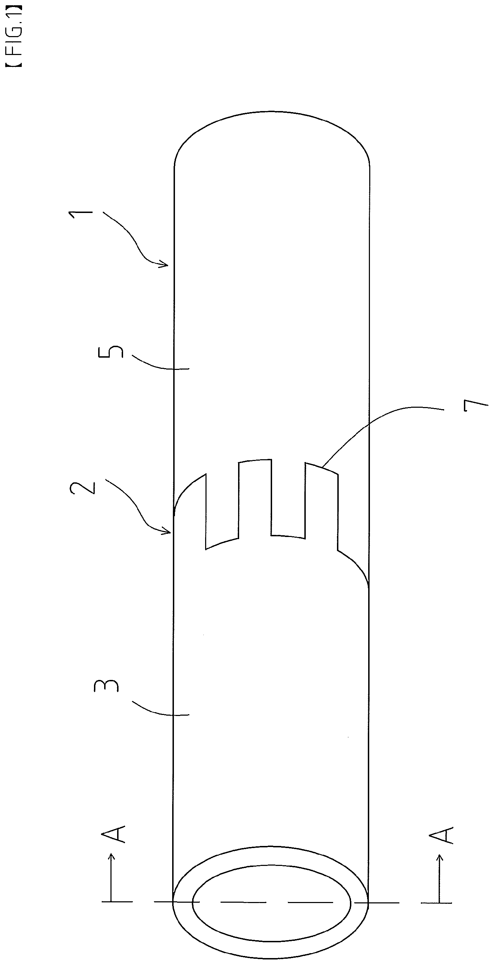

For example, JP 2005 334542 A describes medical tubes used for medical devices such as catheters, in which the medical tubes are joined via a joining area increased at each end of the medical tubes to enhance their joining strength when corresponding ends of the medical tubes are joined (see Fig. 1 and others).

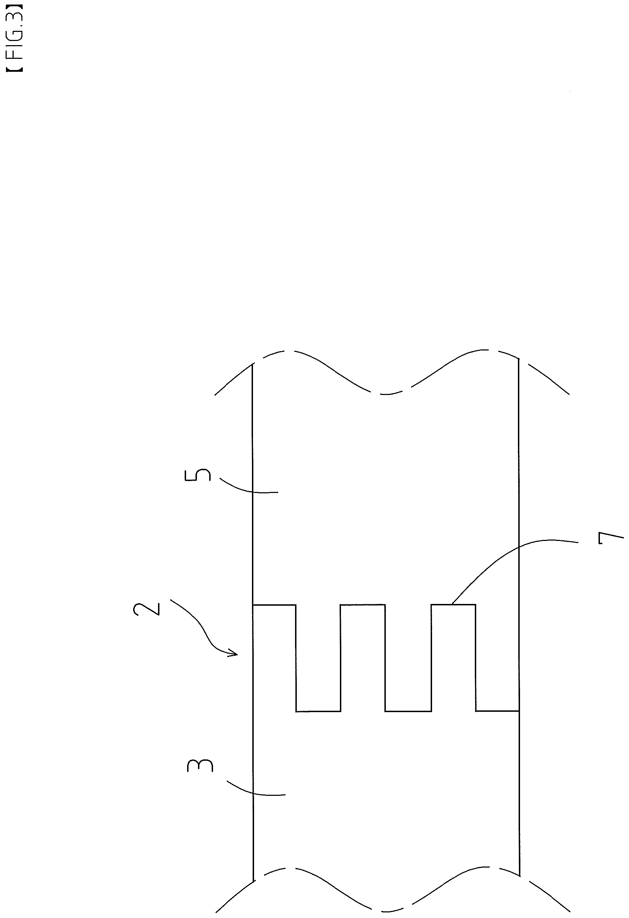

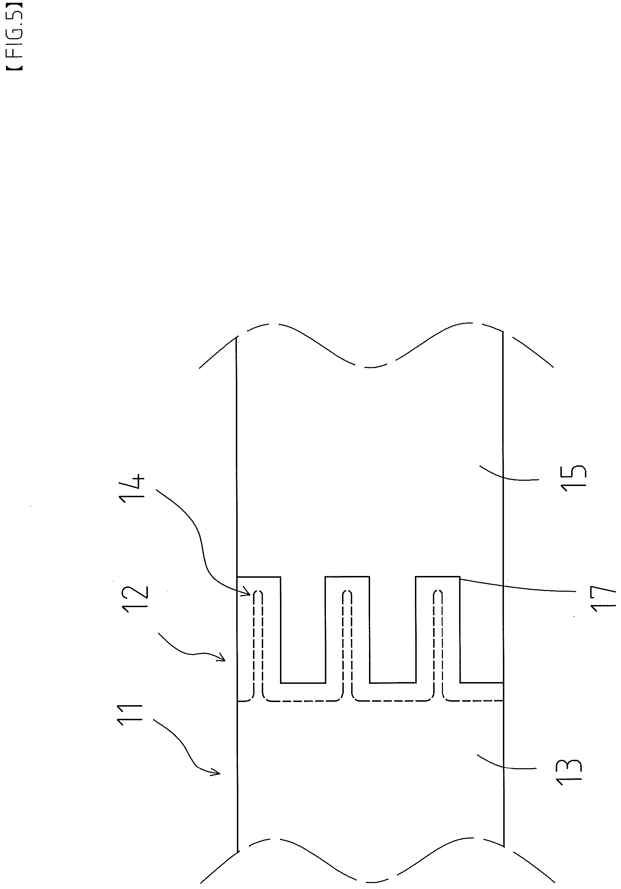

However, in the medical tubes described in JP 2005 334542 A , the joining strength is weak against a pressure in the radial direction. This may result in the following disadvantages: for example, when joined via tapered portions each formed at the end portions (see Fig. 3(b)), the joined tapered portions are susceptible to formation of a crack upon bending the medical tubes; and when joined via stepped portions each formed at the end portions (see Fig. 5), the joined stepped portions are susceptible to formation of a crack upon bending the medical tubes.

Incidentally, WO 97/14466 A1, WO 2014/099899 A1 and WO 2009/125575 A1 each disclose a junction structure between a first tubular member and a second tubular member. An interface between the first tubular member and the second tubular member has an uneven shape in a long axis direction along a circumferential direction in a front view. EP 2135634 A1 discloses a lumen layer consisting of several parts.

US 6 103 037 A discloses a junction structure according to the preamble of claim 1.

US 5 584 821 A discloses a junction structure between nylon plies and a tip polyether block amide (PEBA).

### SUMMARY OF THE INVENTION

### PROBLEMS TO BE SOLVED BY THE INVENTION

The present invention is made to solve the above problems. An object of the present invention is to provide a junction structure in which the joining strength thereof is further improved by adding an enhanced joining strength against a pressure in the radial direction. Another object of the present invention is to provide a catheter having the above junction structure.

### SOLUTIONS TO THE PROBLEMS

The above described technical problem is solved by the subject-matter of claim 1.

Preferred embodiments of the present invention are the subject-matter of dependent claims.

### ADVANTAGEOUS EFFECT OF THE INVENTION

In the junction structure between the first tubular member made of resin and the second tubular member made of resin according to the subject-matter of claim 1, an interface between the first tubular member and the second tubular member has a protrusion-depression shape in the long axis direction along the circumferential direction in a front view, and any one of the first tubular member and the second tubular member is configured to enter into the other tubular member at a region between a radial-direction outer edge and a radial-direction inner edge of the other tubular member when in a cross sectional view along the long axis direction. This can enhance the joining strength between the first tubular member and the second tubular member against a pressure in the radial direction. This can also increase a joining area to enhance the joining strength between the first tubular member and the second tubular member.

According to the junction structure of claim 2, the effect of the subject matter of claim 1 can be provided. In addition, this can prevent projection of a surface of the convex tip from a surface of the tubular member.

Further, the catheter according to claim 3 can benefit from the effect of the first or second aspect of the present invention.

Further, the catheter according to claim 4 can further enhance the joining strength between the first tubular member and the second tubular member.

### BRIEF DESCRIPTION OF DRAWINGS

Fig. 1 shows a perspective view of a junction structure according to the first embodiment of the present invention.
Fig. 2 shows a cross sectional view near the joining region when the junction structure of Fig. 1 is cut in the direction of A-A.
Fig. 3 shows a front view near the joining region of the junction structure according to the first embodiment.
Fig. 4 shows a front view near the joining region in which entering tops are shown in Fig. 3.
Fig. 5 shows a front view near the joining region in which entering tops are shown in a junction structure according to the second embodiment.
Fig. 6 shows a front view near the joining region of a junction structure according to the third embodiment.
Fig. 7 shows a front view near the joining region in which entering tops are shown in Fig. 6.
Fig. 8 shows a front view near the joining region of a junction structure according to the fourth embodiment.
Fig. 9 shows a front view near the joining region in which entering tops are shown in Fig. 8.
Fig. 10 shows the overall view of a catheter according to the fifth embodiment.
Fig. 11 shows a cross sectional view near the joining region of a catheter according to the sixth embodiment.
Fig. 12 shows a cross sectional view near the joining region of a catheter according to the seventh embodiment.

### DESCRIPTION OF EMBODIMENTS

Below, the aforementioned embodiments of the present invention will be described with reference to the drawings.

### (First embodiment)

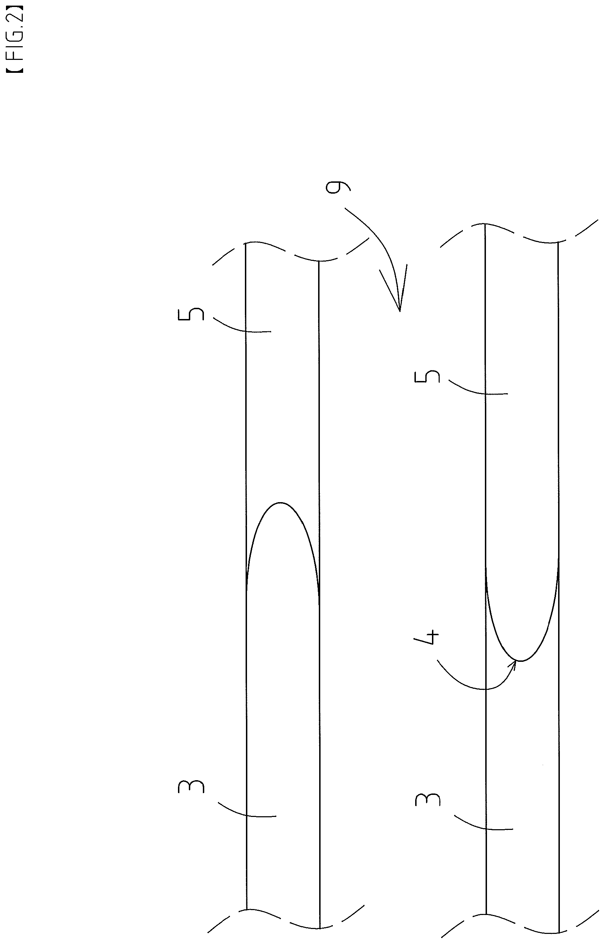

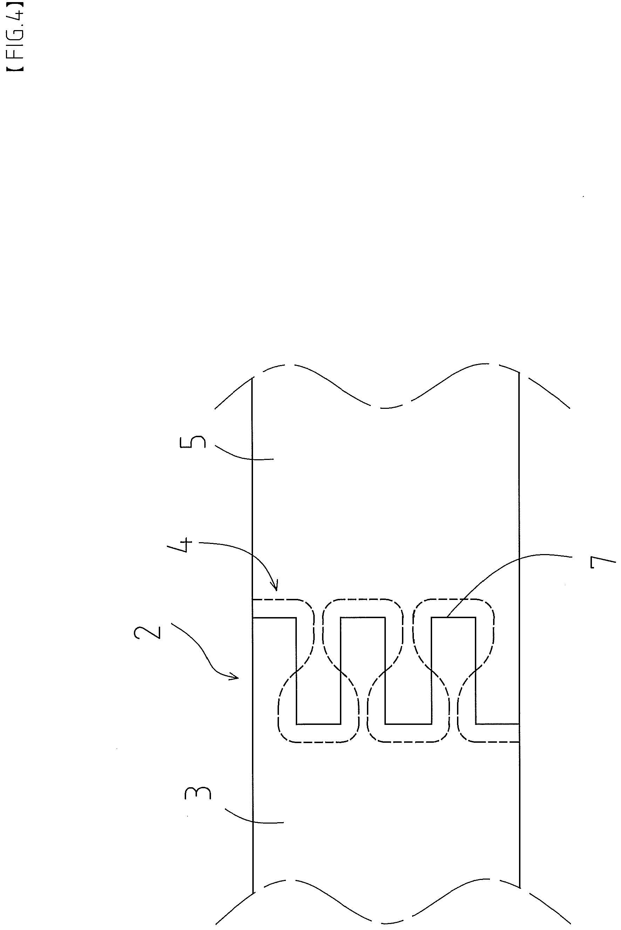

Fig. 1 shows a perspective view of the junction structure according to the first embodiment of the present invention, and Fig. 2 shows a cross sectional view near the joining region when the junction structure of Fig. 1 is cut in the direction of A-A, and Fig. 3 shows a front view near the joining region of the junction structure according to the first embodiment, and Fig. 4 shows a front view near the joining region in which entering tops are shown in Fig. 3.

With reference to Fig. 1, a tubular member 3 made of resin and a tubular member 5 made of resin are joined at a junction structure 1. An interface 7 between the tubular member 3 and the tubular member 5 at a joining region 2 has a protrusion-depression shape in the long axis direction along the circumferential direction of the tubular members.

It is noted that the protrusion-depression shape in this embodiment is approximately rectangular along the circumferential direction of the tubular members as shown in Fig. 1.

Here, the interface 7 between the tubular member 3 and the tubular member 5 is configured to have a protrusion-depression shape in the long axis direction along the circumferential direction of the tubular members in order to increase a joining area between the tubular member 3 and the tubular member 5 as much as possible. This can improve the joining strength between the tubular member 3 and the tubular member 5.

Fig. 2 shows a cross sectional view near the joining region when the junction structure 1 shown in Fig. 1 is cut in the direction of A-A. With reference to Fig. 2, the tubular member 3 and the tubular member 5 each have a inner cavity 9, the inner cavities 9 communicating mutually, and at the interface 7 between the tubular member 3 and the tubular member 5, the tubular member 3 is configured to enter at a region between the radial-direction outer edge and the radial-direction inner edge of the tubular member 5 (a top 4 represents a forefront of the entering portion) in the upper portion of the figure, and the tubular member 5 is configured to enter at a region between the radial-direction outer edge and the radial-direction inner edge of the tubular member 3 (the top 4 represents a forefront of the entering portion) in the lower portion of the figure.

That is, in this embodiment, the tubular member 3 or the tubular member 5 formed to have a convex shape is configured to enter into the other tubular member at a region between the radial-direction outer edge and the radial-direction inner edge of the other tubular member.

Here, one of the tubular member 3 or the tubular member 5 is configured to enter at a region between the radial-direction outer edge and the radial-direction inner edge of the other tubular member in order to improve the joining strength between the tubular member 3 and the tubular member 5 when a force is applied to the interface 7 between the tubular member 3 and the tubular member 5 in the radial direction (the outward or inward direction from a line radially extending from the center of the tubular member, i.e., the vertical direction in Fig. 2).

Further, the tubular member 3 or the tubular member 5 formed to have a convex shape is configured to enter into the other tubular member at a region between the radial-direction outer edge and the radial-direction inner edge of the other tubular member particularly in order to prevent projection of a surface of the convex tip from a surface of the tubular member.

Further, the tubular member 3 or the tubular member 5 formed to have a convex shape is also configured to enter into the other tubular member at a region between the radial-direction outer edge and the radial-direction inner edge of the other tubular member as described above in order to increase a joining area between the tubular member 3 and the tubular member 5 as much as possible. This can improve the joining strength between the tubular member 3 and the tubular member 5.

Fig. 3 shows a front view near the joining region of the junction structure according to the first embodiment, and Fig. 4 shows a front view near the joining region in which the configuration of the entering top 4 drawn in dotted lines is added to Fig. 3.

As clearly shown in Fig. 4, the top 4 of the tubular member 3 or the tubular member 5 formed to have a convex shape enters into the other tubular member.

It is noted that the entering resins are interchanged at the middle position of a laterally adjacent protrusion-depression in Fig. 4. That is, the resin of the tubular member 5 enters into the resin of the tubular member 3 in the left-side region relative to the middle position while the resin of the tubular member 3 enters into the resin of the tubular member 5 in the right-side region relative to the middle position.

### (Second embodiment)

Below, the second embodiment of the present invention will be described. Fig. 5 shows a front view near the joining region in which an entering top 14 is shown in a junction structure 11 according to the second embodiment.

With reference to Fig. 5, a tubular member 13 made of resin and a tubular member 15 made of resin are joined at the junction structure 11. An interface 17 between the tubular member 13 and the tubular member 15 at a joining region 12 has a protrusion-depression shape in the long axis direction along the circumferential direction of the tubular members.

It is noted that the protrusion-depression shape in this embodiment is also approximately rectangular along the circumferential direction of the tubular members as in the junction structure 1 according to the first embodiment.

Therefore, the interface 17 between the tubular member 13 and the tubular member 15 at the joining region 12 also has a protrusion-depression shape in the long axis direction along the circumferential direction in the junction structure 11 according to this embodiment. This can increase a joining area to enhance the joining strength between the first tubular member and the second tubular member.

It is noted that in the junction structure 11 according to this embodiment, the tubular member 15 enters at a region between the radial-direction outer edge and the radial-direction inner edge of the tubular member 13 entirely in the circumferential direction. That is, in the junction structure 11 according to this embodiment, the top 14 of the tubular member 15 formed to have a convex shape enters at a region between the radial-direction outer edge and the radial-direction inner edge of the tubular member 13 entirely in the circumferential direction as shown in Fig. 5.

Therefore, again in the junction structure 11 according to this embodiment, the joining strength between the tubular member 13 and the tubular member 15 can be improved even when a force is applied to the interface 17 between the tubular member 13 and the tubular member 15 in the radial direction.

Further, the top 14 of the tubular member 15 formed to have a convex shape enters at a region between the radial-direction outer edge and the radial-direction inner edge of the tubular member 13 entirely in the circumferential direction. This can also increase a joining area between the tubular member 13 and the tubular member 15 as much as possible to improve the joining strength between the tubular member 13 and the tubular member 15.

### (Third embodiment)

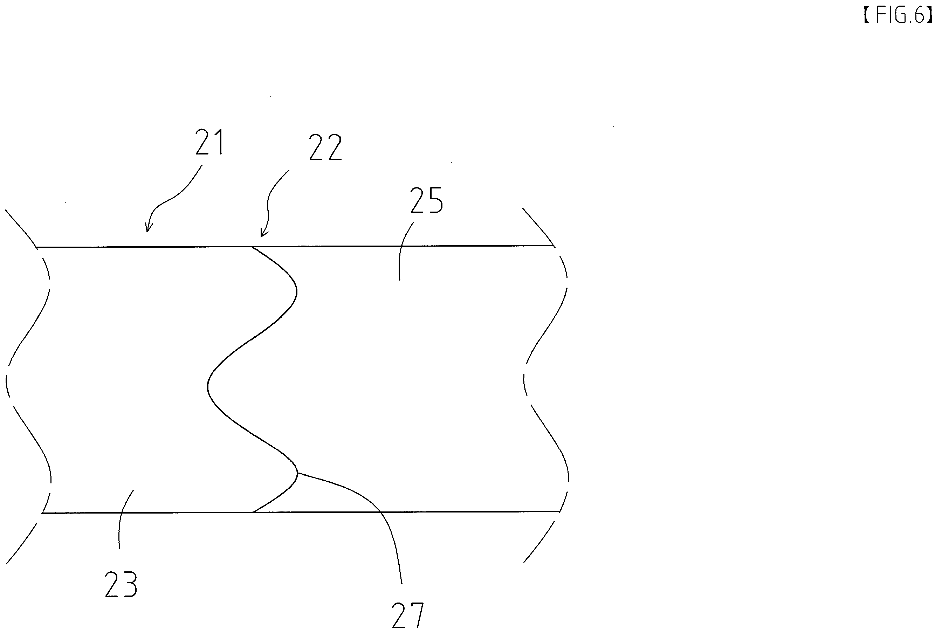

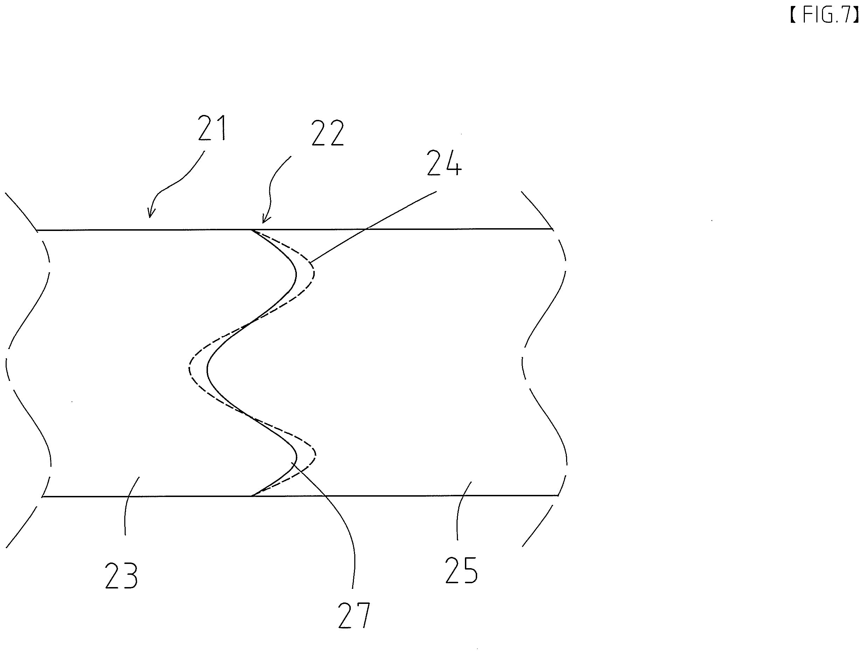

Below, the third embodiment of the present invention will be described. Fig. 6 shows a front view near the joining region of a junction structure 21 according to the third embodiment, and Fig. 7 shows a front view near the joining region in which an entering top 24 drawn in dotted lines is added to Fig. 6.

With reference to Fig. 6, a tubular member 23 made of resin and a tubular member 25 made of resin are joined at the junction structure 21. An interface 27 between the tubular member 23 and the tubular member 25 at a joining region 22 has a protrusion-depression shape in the long axis direction along the circumferential direction of the tubular members.

It is noted that the protrusion-depression shape in this embodiment is corrugated along the circumferential direction of the tubular members as shown in Fig. 6.

Here, the interface 27 between the tubular member 23 and the tubular member 25 is configured to have a protrusion-depression shape in the long axis direction along the circumferential direction of the tubular members in order to increase a joining area between the tubular member 23 and the tubular member 25 as much as possible as in the first and second embodiments. This can improve the joining strength between the tubular member 23 and the tubular member 25.

Again, in this embodiment, the interface between the tubular member 23 and the tubular member 25 has a portion in which the tubular member 23 or the tubular member 25 is configured to enter at a region between the radial-direction outer edge and the radial-direction inner edge of the other tubular member in a cross section (the top 24 represents a forefront of the entering portion).

That is, in this embodiment, the tubular member 23 or the tubular member 25 formed to have a convex shape is configured to enter into the other tubular member at a region between the radial-direction outer edge and the radial-direction inner edge of the other tubular member.

Here, one of the tubular member 23 or the tubular member 25 is configured to enter at a region between the radial-direction outer edge and the radial-direction inner edge of the other tubular member in order to improve the joining strength between the tubular member 23 and the tubular member 25 when a force is applied to the interface 27 between the tubular member 23 and the tubular member 25 in the radial direction as in the first and second embodiments.

Further, the tubular member 23 or the tubular member 25 formed to have a convex shape is configured to enter into the other tubular member at a region other than the both edges of the other tubular member particularly in order to prevent projection of a surface of the convex tip from a surface of the tubular member.

Further, the tubular member 23 or the tubular member 25 formed to have a convex shape is configured to enter into the other tubular member at a region other than the both edges of the other tubular member particularly in order to increase a joining area between the tubular member 23 and the tubular member 25 as much as possible. This can also improve the joining strength between the tubular member 23 and the tubular member 25.

As clearly shown in Fig. 7, the top 24 of the tubular member 23 or the tubular member 25 formed to have a convex shape enters into the other tubular member.

It is noted that the entering resins are interchanged at the middle position of a laterally adjacent protrusion-depression in Fig. 7. That is, the resin of the tubular member 25 enters into the resin of the tubular member 23 in the left-side region relative to the middle position while the resin of the tubular member 23 enters into the resin of the tubular member 25 in the right-side region relative to the middle position.

### (Fourth embodiment)

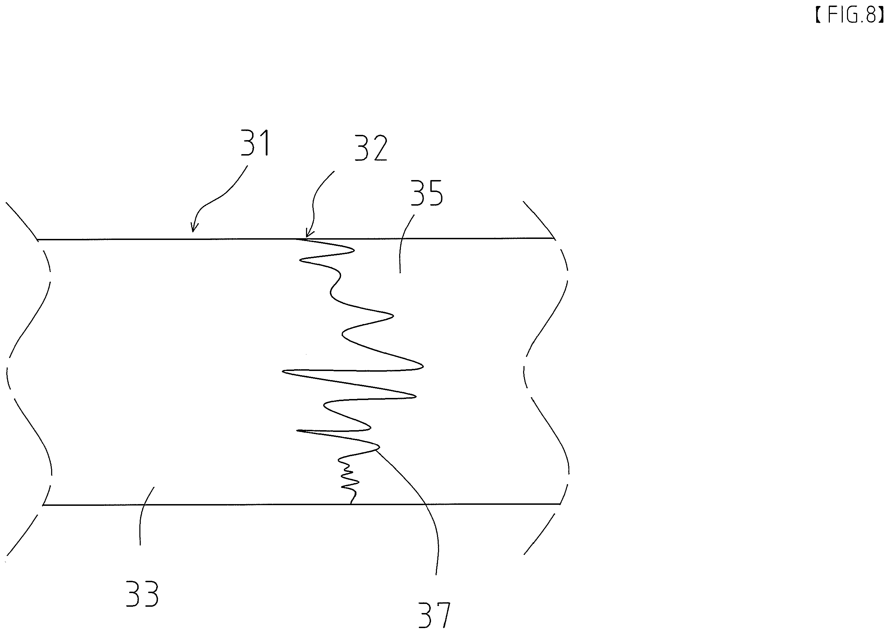

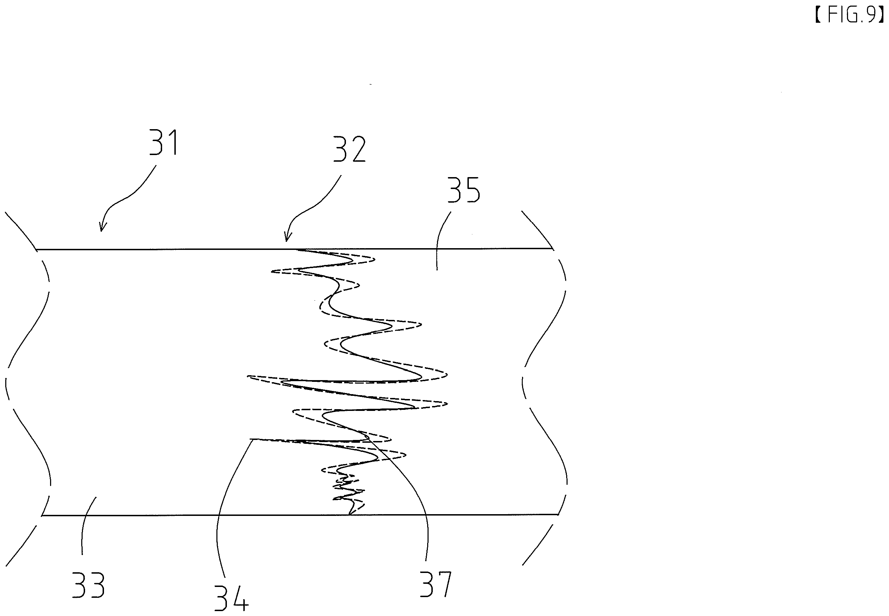

Below, the fourth embodiment of the present invention will be described. Fig. 8 shows a front view near the joining region of a junction structure 31 according to the fourth embodiment, and Fig. 9 shows a front view near the joining region in which an entering top 34 is shown in Fig. 8.

With reference to Fig. 8, a tubular member 33 made of resin and a tubular member 35 made of resin are joined at the junction structure 31. An interface 37 between the tubular member 33 and the tubular member 35 at a joining region 32 has a protrusion-depression shape in the long axis direction along the circumferential direction of the tubular members.

Here, the interface 37 between the tubular member 33 and the tubular member 35 is configured to have a protrusion-depression shape in the long axis direction along the circumferential direction of the tubular members in order to increase a joining area between the tubular member 33 and the tubular member 35 as much as possible as in the first to third embodiments. This can improve the joining strength between the tubular member 33 and the tubular member 35.

Again, in this embodiment, the interface between the tubular member 33 and the tubular member 35 has a portion in which the tubular member 33 or the tubular member 35 enters at a region between the radial-direction outer edge and the radial-direction inner edge of the tubular member of the other tubular member in a cross section (the top 34 represents a forefront of the entering portion).

That is, in this embodiment, the tubular member 33 or the tubular member 35 formed to have a convex shape is configured to enter into the other tubular member at a region between the radial-direction outer edge and the radial-direction inner edge of the other tubular member.

Here, as in the first to third embodiments, one of the tubular member 33 or the tubular member 35 is configured to enter at a region between the radial-direction outer edge and the radial-direction inner edge of the other tubular member in order to improve the joining strength between the tubular member 33 and the tubular member 35 when a force is applied to the interface 37 between the tubular member 33 and the tubular member 35 in the radial direction.

Further, the tubular member 33 or the tubular member 35 formed to have a convex shape is configured to enter into the other tubular member at a region between the radial-direction outer edge and the radial-direction inner edge of the other tubular member particularly in order to prevent projection of a surface of the convex tip from a surface of the tubular member.

Further, one of the tubular member 33 or the tubular member 35 is configured to enter at a region between the radial-direction outer edge and the radial-direction inner edge of the other tubular member in order to increase a joining area between the tubular member 33 and the tubular member 35 as much as possible. This can also improve the joining strength between the tubular member 33 and the tubular member 35.

As clearly shown in Fig. 9, the top 34 of the tubular member 33 or the tubular member 35 formed to have a convex shape enters into the other tubular member.

It is noted that the entering resins are interchanged at the middle position of a laterally adjacent protrusion-depression in Fig. 9. That is, the resin of the tubular member 35 enters into the resin of the tubular member 33 in the left-side region relative to the middle position while the resin of the tubular member 33 enters into the resin of the tubular member 35 in the right-side region relative to the middle position.

### (Fifth embodiment)

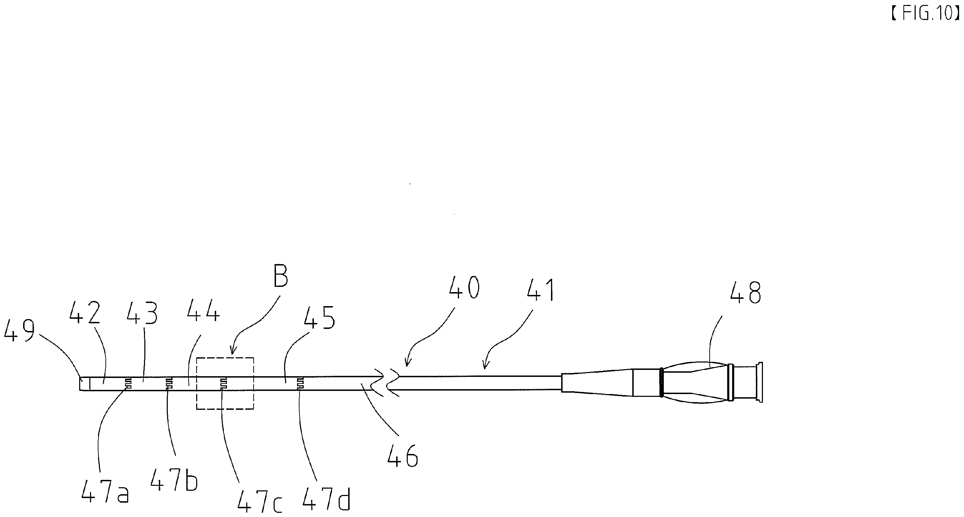

Below, the fifth embodiment of the present invention will be described. Fig. 10 shows the overall view of a catheter 41 according to the fifth embodiment.

With reference to Fig. 10, the catheter 41 includes a catheter tube body 40, a front end tip 49 connected to the front end of the catheter tube body 40, and a connector 48 connected to the base end of the catheter tube body 40.

The catheter tube body 40 is configured such that multiple tubular members made of different resin materials are joined so that the catheter tube body 40 becomes increasingly more flexible toward the front end from the base end. In this embodiment, the catheter tube body 40 includes a tubular member 46, a tubular member 45, a tubular member 44, a tubular member 43, and a tubular member 42 in this order from the base end.

That is, in this embodiment, the resin of the tubular member 46 is hardest, and the resins are softer in the order of the tubular member 45, the tubular member 44, the tubular member 43, and the tubular member 42. The resin of the tubular member 42 is softest.

Further, the catheter tube body 40 according to this embodiment, in which the junction structures 1 according to the first embodiment are used at 4 positions, includes an interface 47d, an interface 47c, an interface 47b, and an interface 47a in this order from the base end.

According to the catheter 41 according to this embodiment, the catheter tube body 40 includes the junction structures 1. This can further improve the joining strength between the tubular member 42 and the tubular member 43, the joining strength between the tubular member 43 and the tubular member 44, the joining strength between the tubular member 44 and the tubular member 45, and the joining strength between the tubular member 45 and the tubular member 46 by adding an improved joining strength against a pressure in the radial direction. This, in turn, can improve the joining strength of the catheter tube body 40.

### (Sixth embodiment)

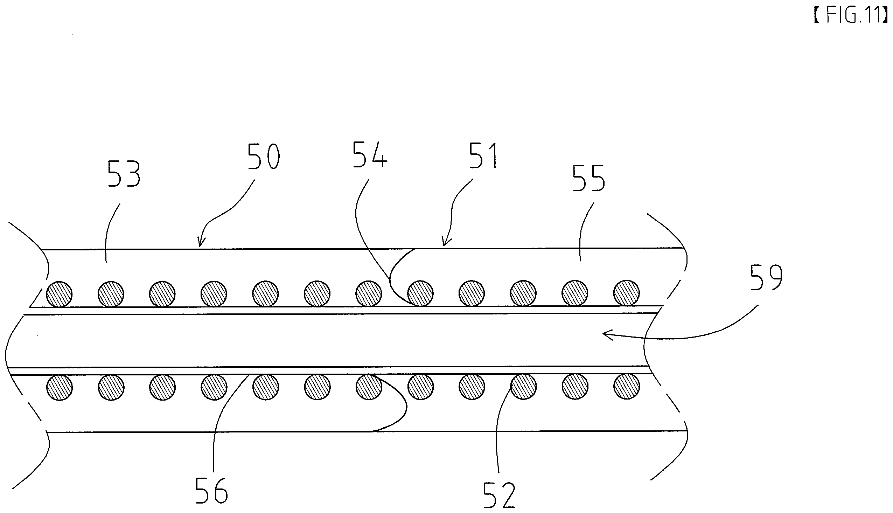

Below, the sixth embodiment of the present invention will be described. Fig. 11 shows a cross sectional view near the joining region (corresponding to the B region in Fig. 10) in a catheter 51 according to the sixth embodiment.

It is noted that the catheter 51 according to this embodiment includes a catheter tube body 50, a front end tip (not shown) connected to the front end of the catheter tube body 50, and a connector (not shown) connected to the base end of the catheter tube body 50 as in the catheter 41 according to the fifth embodiment.

With reference to Fig. 11, the catheter tube body 50 according to this embodiment, unlike the catheter tube 40 according to the fifth embodiment, is not formed with a monolayer tubular member, but includes an inner layer 56, a coil body 52 wound around the outer periphery of the inner layer 56, and outer layers 53 and 55 covering the outer peripheries of the inner layer 56 and the coil body 52, and has an inner cavity 59.

The inner layer 56, the coil body 54, and the outer layers 53 and 55 are each tubular members, and the outer layers 53 and 55 have the junction structure 1 according to Embodiment 1.

Here, the outer layers 53 and 55 are formed such that a top 54 of one of the tubular members enters at a region between the radial-direction outer edge and the radial-direction inner edge of the other tubular member, and the radial-direction inner edge of the outer layer 53 or the outer layer 55 enters between the coil body 52 and the inner layer 56.

Further, in the catheter tube body 50, multiple tubular members made of different resin materials are joined so that the catheter tube body 50 becomes increasingly more flexible toward the front end from the base end. In this embodiment, four tubular members are joined as in the catheter tube 40 according to the fifth embodiment.

It is noted that among the four tubular members, the tubular member 55 in the base end side, and the tubular member 53 in the front end side are shown in Fig. 11.

The catheter 51 according to this embodiment includes an inner layer 56, a coil body 52 wound around the outer periphery of the inner layer 56, and outer layers 53 and 55 covering the outer peripheries of the inner layer 56 and the coil body 52, in which the outer layers 53 and 55 are formed such that one of the outer layers enters at a region between the radial-direction outer edge and the radial-direction inner edge of the other outer layer in a cross sectional view, and the inner edge of the outer layer 53 or the outer layer 55 enters between the coil body and the inner layer 56. This can further enhance the joining strength between the outer layer 53 and the outer layer 55, which in turn can improve the joining strength of the catheter tube body 50.

### (Seventh embodiment)

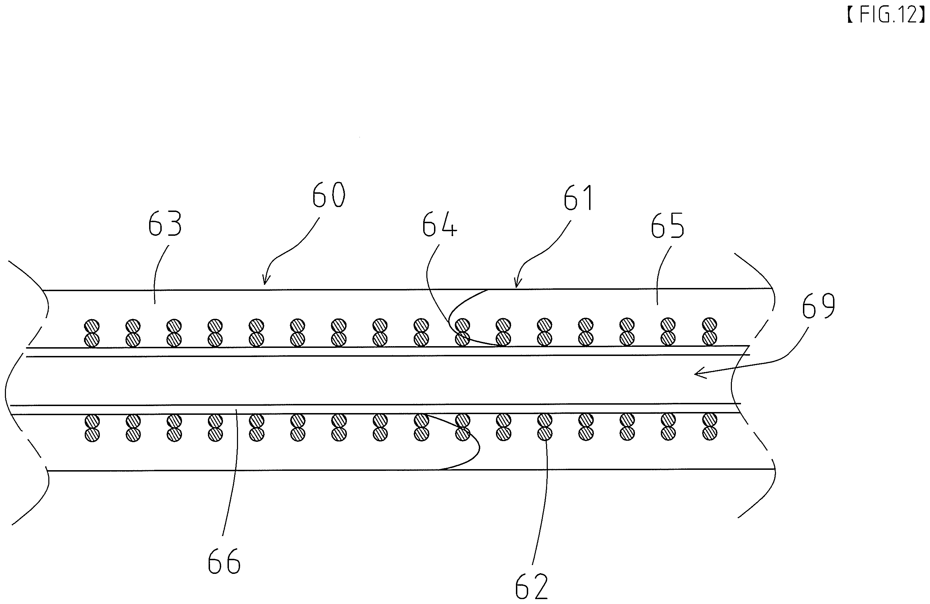

Below, the seventh embodiment of the present invention will be described. Fig. 12 shows a cross sectional view near the joining region (corresponding to the B region in Fig. 10) in a catheter 61 according to the seventh embodiment.

It is noted that the catheter 61 according to this embodiment includes a catheter tube body 60, a front end tip (not shown) connected to the front end of the catheter tube body 60, and a connector (not shown) connected to the base end of the catheter tube body 60 as in the catheter 41 according to the fifth embodiment.

With reference to Fig. 12, the catheter tube body 60, unlike the catheter tube 40 according to the fifth embodiment, is not formed with a monolayer tubular member, but includes an inner layer 66, a braid 62 wound around the outer periphery of the inner layer 66, and outer layers 63 and 65 covering the outer peripheries of the inner layer 66 and the braid 62, and has an inner cavity 69.

The inner layer 66, the braid 62, and the outer layers 63 and 65 are each tubular members, and the outer layers 63 and 65 have the junction structure 1 according to Embodiment 1.

Here, the outer layers 63 and 65 are formed such that a top 64 of one of the tubular members enters at a region between the radial-direction outer edge and the radial-direction inner edge of the other tubular member, and the radial-direction inner edge of the outer layer 63 or the outer layer 65 enters between the braid 62 and the inner layer 66.

Further, in the catheter tube body 60, multiple tubular members made of different resin materials are joined so that the catheter tube body 60 becomes increasingly more flexible toward the front end from the base end. In this embodiment, four tubular members are joined as in the catheter tube 41 according to the fifth embodiment.

It is noted that among the four tubular members, the tubular member 65 in the base end side, and the tubular member 63 in the front end side are shown in Fig. 12.

The catheter 61 according to this embodiment includes an inner layer 66, a braid 62 wound around the outer periphery of the inner layer 66, and outer layers 63 and 65 covering the outer peripheries of the inner layer 66 and the braid 62, in which the outer layers 63 and 65 are configured such that one of the outer layers enters at a region between the radial-direction outer edge and the radial-direction inner edge of the other outer layer in a cross sectional view, and the radial-direction inner edge of the outer layer 63 or the outer layer 65 enters between the braid 62 and the inner layer 66. This can further enhance the joining strength between the outer layer 63 and the outer layer 65, which in turn can improve the joining strength of the catheter tube body 60.

For example, the catheters according to the fifth to seventh embodiments described above include the junction structures 1 according to the first embodiment, but the junction structure 11 according to the second embodiment, the junction structure 21 according to the third embodiment, or the junction structure 31 according to the fourth embodiment may be used. In these cases, the catheter tube 40 according to the fifth embodiment, the catheter tube 50 according to the sixth embodiment, and the catheter tube 60 according to the seventh embodiment can benefit from the aforementioned effects expected from the junction structures used.

Further, the catheters according to the fifth to seventh embodiments described above are configured to have four joined tubular members. However, the number of tubular members is not limited to four, and any number of tubular members may be used.

### DESCRIPTION OF SYMBOLS

1, 11, 21, 31 Junction structure
2, 22, 32 Joining region
3, 5, 13, 15, 23, 25, 33, 35, 42, 43, 44, 45, 46 Tubular member
4, 14, 24, 34, 54, 64 Top
7, 17, 27, 37, 47a, 47b, 47c, 47d Interface
9, 59, 69 Inner cavity
40, 50, 60 Catheter tube
41,51,61 Catheter
48 Connector
49 Front end tip
52 Coil body
53, 55, 63, 65 Outer layer
56, 66 Inner layer
62 Braid

## Claims

1. A junction structure (1; 11; 21; 31) between a first tubular member (3, 13, 23, 33, 42-45) made of resin and a second tubular member (5; 15; 25; 35; 43-46) made of resin,
wherein an interface (7; 17; 27; 37; 47a, 47b, 47c, 47d) between the first tubular member (3, 13, 23, 33, 42-45) and the second tubular member (5; 15; 25; 35; 43-46) has a protrusion-depression shape in a long axis direction, protrusions and depressions of the protrusion-depression shape being laterally adjacent to each other along a circumferential direction in a front view, **characterised in that**
any one of the first tubular member (3, 13, 23, 33, 42-45) and the second tubular member (5; 15; 25; 35; 43-46) is configured to enter into the other tubular member at a region between a radial-direction outer edge and a radial-direction inner edge of the other tubular member in a cross sectional view along the long axis direction and is formed to have a convex shape in the cross sectional view along the long axis direction,.

2. The junction structure (1; 11; 21; 31) according to claim 1, wherein any one of the first tubular member (3, 13, 23, 33, 42-45) and the second tubular member (5; 15; 25; 35; 43-46) is configured to enter into the other tubular member at the entire region having the protrusion-depression shape.

3. A catheter (41; 51; 61) having the junction structure (1; 11; 21; 31) according to claim 1 or 2.

4. The catheter (51; 61) according to claim 3, comprising
an inner layer (56; 66),
a braid (62) or a coil body (54) wound around the outer periphery of the inner layer (56; 66), and
outer layers (53, 55; 63, 65) covering the outer peripheries of the inner layer (56; 66) and the braid (62) or the coil body (54), wherein:
the outer layers (53, 55; 63, 65) have the junction structure (1; 11; 21; 31) according to claim 1 or 2, and
the radial-direction inner edge of the other tubular member receiving intrusion at a region between the both edges enters between the braid (62) or the coil body (54) and the inner layer (56; 66).

## Patentansprüche

1. Verbindungsstruktur (1; 11; 21; 31) zwischen einem ersten röhrenförmigen Element (3, 13, 23, 33, 42-45) aus Harz und einem zweiten röhrenförmigen Element (5; 15; 25; 35; 43-46) aus Harz,
wobei eine Grenzfläche (7; 17; 27; 37; 47a, 47b, 47c, 47d) zwischen dem ersten röhrenförmigen Element (3, 13, 23, 33, 42-45) und dem zweiten röhrenförmigen Element (5; 15; 25; 35; 43-46) in einer Längsachsenrichtung eine Vorsprung-Vertiefungs-Form aufweist, wobei Vorsprünge und Vertiefungen der Vorsprung-Vertiefungs-Form entlang einer Umfangsrichtung in einer Vorderansicht seitlich benachbart zueinander vorliegen, **dadurch gekennzeichnet, dass**
eines von dem ersten rohrförmigen Element (3, 13, 23, 33, 42-45) und dem zweiten rohrförmigen Element (5; 15; 25; 35; 43-46) konfiguriert ist, dass es in das andere rohrförmige Element in einem Bereich zwischen einer Außenkante in radialer Richtung und einer Innenkante in radialer Richtung des anderen rohrförmigen Elements in einer Querschnittsansicht entlang der Längsachsenrichtung eintritt und ausgebildet ist, dass es in der Querschnittsansicht entlang der Längsachsenrichtung eine konvexe Form aufweist.

2. Verbindungsstruktur (1; 11; 21; 31) nach Anspruch 1, wobei eines von dem ersten rohrförmigen Element (3, 13, 23, 33, 42-45) und dem zweiten rohrförmigen Element (5; 15; 25; 35; 43-46) konfiguriert ist, dass es in das andere röhrenförmige Element an dem gesamten Bereich mit der Vorsprung-Vertiefungs-Form eintritt.

3. Katheter (41; 51; 61) mit der Verbindungsstruktur (1; 11; 21; 31) nach Anspruch 1 oder 2.

4. Katheter (51; 61) nach Anspruch 3, umfassend
eine Innenschicht (56; 66),
ein Geflecht (62) oder einen Spulenkörper (54), das bzw. der um den Außenumfang der Innenschicht (56; 66) gewickelt ist, und
Außenschichten (53, 55; 63, 65), die die Außenumfänge der Innenschicht (56; 66) und des Geflechts (62) oder des Spulenkörpers (54) bedecken, wobei:
die Außenschichten (53, 55; 63, 65) die Verbindungsstruktur (1; 11; 21; 31) nach Anspruch 1 oder 2 aufweisen, und
die Innenkante in radialer Richtung des anderen rohrförmigen Elements, der eine Intrusion an einem Bereich zwischen den beiden Kanten aufnimmt, zwischen dem Geflecht (62) oder dem Spulenkörper (54) und der Innenschicht (56; 66) eintritt.

## Revendications

1. Structure de jonction (1 ; 11 ; 21 ; 31) entre un premier élément tubulaire (3, 13, 23, 33, 42-45) en résine et un second élément tubulaire (5 ; 15 ; 25 ; 35 ; 43-46) en résine,
dans laquelle une interface (7 ; 17 ; 27 ; 37 ; 47a, 47b, 47c, 47d) comprise entre le premier élément tubulaire (3, 13, 23, 33, 42-45) et le second élément tubulaire (5 ; 15 ; 25 ; 35 ; 43-46) présente une forme en saillie-creux dans une direction de l'axe longitudinal, des saillies et des creux de la forme en saillie-creux étant latéralement adjacents les unes aux autres selon une direction circonférentielle dans une vue de face, **caractérisée en ce que**
l'un quelconque parmi le premier élément tubulaire (3, 13, 23, 33, 42-45) et le second élément tubulaire (5 ; 15 ; 25 ; 35 ; 43-46) est configuré pour pénétrer dans l'autre élément tubulaire au niveau d'une zone comprise entre un bord extérieur de direction radiale et un bord intérieur de direction radiale de l'autre élément tubulaire dans une vue en coupe transversale selon la direction de l'axe longitudinal et est formé de manière à présenter une forme convexe dans la vue en coupe transversale selon la direction de l'axe longitudinal.

2. Structure de jonction (1 ; 11 ; 21 ; 31) selon la revendication 1, dans laquelle l'un quelconque parmi le premier élément tubulaire (3, 13, 23, 33, 42-45) et le second élément tubulaire (5 ; 15 ; 25 ; 35 ; 43-46) est configuré pour pénétrer dans l'autre élément tubulaire au niveau de la zone entière présentant une forme en saillie-creux.

3. Cathéter (41 ; 51 ; 61) présentant la structure de jonction (1 ; 11 ; 21 ; 31) selon la revendication 1 ou 2.

4. Cathéter (51 ; 61) selon la revendication 3, comprenant
une couche intérieure (56 ; 66),
un tressage (62) ou un corps de bobine (54) enroulé autour de la périphérie extérieure de la couche intérieure (56 ; 66), et
des couches extérieures (53, 55 ; 63, 65) recouvrant les périphéries extérieures de la couche intérieure (56 ; 66) et le tressage (62) ou le corps de bobine (54), dans lequel :
les couches extérieures (53, 55 ; 63, 65) présentent la structure de jonction (1 ; 11 ; 21 ; 31) selon la revendication 1 ou 2, et
le bord intérieur de direction radiale de l'autre élément tubulaire recevant une intrusion au niveau d'une zone comprise entre les deux bords pénètre entre le tressage (62) ou le corps de bobine (54) et la couche intérieure (56 ; 66).
